# EUROPEAN PATENT APPLICATION

(11) **EP 2 995 938 A1**
(43) Date of publication of application: **16.03.2016**
(21) Application number: 14003187.3
(22) Date of filing: 15.09.2014
(51) Int. Cl.: G01N 27/12, G01N 33/00, F02D 41/14

(54) **Integrated metal oxide chemical sensor**

(71) Applicant: Sensirion AG, 8712 Stäfa (CH)
(72) Inventor: Röck, Frank, CH-8712 Stäfa (CH); Bürgi, Lukas, CH-8712 Stäfa (CH); Höhne, Felix, CH-8712 Stäfa (CH)
(74) Representative: Toleti, Martin

(57) **Abstract**

A chemical sensor (10) is described with at least one layer of a metal oxide (11) arranged between two electrodes (16), one or more heating elements (15) arranged to heat the layer of metal oxide to a predefined temperature or in accordance to a predefined temperature profile, and a control unit (17) for controlling the heating elements, wherein the control unit and the heating elements are designed to heat upon initialisation of a measurement the metal oxide layer such that the layer temperature follows a temperature profile including an activation period during which the layer temperature is above a measurement temperature prior to a measurement period during which the layer temperature is at the measurement temperature, and wherein the control unit derives a concentration value of a gas compound from the values of a parameter related to the resistance of the metal oxide layer as registered during the measurement period.

## Description

### FIELD OF THE INVENTION

The present invention relates to a chemical sensor, particularly a gas sensor using metal oxide, applicable for purposes such as the measurement of breath components.

### BACKGROUND OF THE INVENTION

Metal-oxide sensors are based on the concept that gaseous analytes interact with the metal oxide layer at elevated temperatures of the sensitive layer in the range of more than 100° Celsius, and specifically between 250°C and 350°Celsius. As a result of the catalytic reaction, the conductivity of the sensitive layer may change which change can be measured. Hence, such chemical sensors are also denoted as high temperature chemoresistors for the reason that a chemical property of the analyte is converted into an electrical resistance at high temperatures of the sensitive layer.

A chemical sensor can be a gas sensor for detecting one or more substances in a gas, and specifically in the air surrounding a portable multi-purpose electronic device such a mobile phone or tablet computer. Hence, in a sample application it may be of interest to identify, if such air may contain analytes the chemical sensor is tuned to detect. Specific applications may include the detection of toxic gases, the detection of ethanol, sulphide compounds or other components in a user's breath, or the detection of other substances.

It is also known to arrange such chemical sensors inside a housing of a portable electronic device. An opening may be provided in the housing for exposing the chemical sensor to a fluid to be analyzed.

Generally speaking such chemical sensors have to operate under several constraints. Firstly, they have to show a high sensitivity with respect to the analyte. Secondly, a good chemical sensor has also to show a low cross sensitivity so that the analyte can be detected even in the presence of other gases. A third possible constraint can be the response time of the sensor with a small response time enabling faster measurements.

These general constraints become compounded with other problems when integrating such a sensor within the narrow confines of a modern day portable device. Typically for such devices only a very limited volume is acceded to additional sensors outside the core functionality of the device such as wireless voice or data communication, display, speaker, processors and battery. This means that the real overall dimensions of the sensor, its associated circuitry for control and readout have to be within or close to the sub-millimeter range. Further, the sensor within the portable device has to operate within the confines set by the power available in such a device.

Examples of the known methods to operate chemical sensors are described in the European patent application EP 2642289 or in the published United States patent application no. 2012/0297860. Examples of commercially available sensors are the gas sensor SB-EN3 from FIS Inc. Hyogo, Japan for mouth odour level monitoring. The SB-EN3 is a tin dioxide semiconductor gas sensor of a mini bead type with a heater coil and an electrode wire embedded in the element or the TGS 825 sensor for hydrogen sulfide of Figaro Engineering Inc. Osaka, Japan, with a sensing element of SnO2 sintered to form a thick film on the surface of an alumina ceramic tube which contains an internal heater.

In the light of the above, it is therefore seen as an object of the invention to improve the methods of operating chemical sensors using metal oxide films, particularly for integrated sensors suitable for mounting into the housing of portable multi-purpose devices.

### SUMMARY OF THE INVENTION

Hence, according to a first aspect of the invention, there is provided a chemical sensor comprising at least one layer of a metal oxide arranged between two electrodes, one or more heating elements arranged to heat the layer of metal oxide, and a control unit for controlling the one or more heating elements, wherein the control unit is adapted to control the one or more heating elements to heat upon initialisation of a measurement the metal oxide layer according to a temperature profile including an activation period during which the layer temperature is above a measurement temperature prior to a measurement period during which the layer temperature is at the measurement temperature, and wherein the control unit is adapted to derive a concentration value of a gas compound from the values of a parameter related to a resistance of the metal oxide layer as registered during the measurement period.

In a preferred embodiment, the metal oxide layer, also referred to as sensing layer, is heated according to the temperature profile which may be stored in a memory of the chemical sensor in form of a heating pattern. In a first variant, the heating may be effected by the control unit according to the heating pattern in an open loop mode. In another embodiment, the sensor device comprises a temperature sensor for monitoring the temperature of the metal oxide layer, which temperature is also referred to as layer temperature. Here, the heating may be effected by the control unit according to the heating pattern and the measured layer temperature in a closed loop mode.

It should be noted that each measurement comprises a preheat step followed by a measurement step which are initiated as a single measurement. As a unit of preheat step and measurement step is initiated upon a system or user request to perform a measurement, the sequence of subsequent preheat steps and measurement steps seen as temperature profile is aperiodic.

The unit of preheat step and measurement step also renders the sensor distinct from systems with a sensor refresh or outgassing step as such steps are typically not performed as a unit linked with the measurement step but rather as a periodic step or as step linked to general usage/degree of sensing layer poisoning of the sensor.

It should be further noted that a valid measurement can be performed solely during the measurement period and that a measurement of resistance values during the activation period is optional for the above sensor.

It was found that the above sensor and the related method according to which the sensor is operated improves the cross-sensitivity of the measurement particularly of sulfur compounds such as hydrogen sulfide, carbon monoxide, or methane, in the presence of for example ethanol or humidity (H20). This reduced cross-sensitivity is particularly advantageous for the measurement of breath components.

In yet another variant of the invention the activation period and the following measurement period take place in the time window of less than one minute, as it was found that exposure times to hydrogen sulfide of around 30 seconds can be sufficient to distinguish between concentrations of one hundred ppb, 250 ppb, and 600 ppb.

A maximum temperature of the metal oxide layer during the activation period is best between 200°C and 400°C, preferably in the range between 250°C and 350°C.

A maximum temperature of the metal oxide layer during the measurement period is best between room temperature and 200°C, e.g., between 0°C and 200°C, more preferably in the range between 50°C and 150°C, where for example the sensitivity to hydrogen sulfide is high while the cross sensitivity to ethanol and/or humidity is significantly decreased.

A difference between the maximum temperature of the metal oxide layer during the activation period and the maximum temperature of the metal oxide layer during the measurement period is at least 50°C, more preferably at least 100°C.

In a preferred variant, wherein the temperature profile is such that the layer temperature is not significantly below the measurement temperature between the end of the activation period and the beginning of the measurement period, wherein significantly below is defined as the measurement temperature minus 10% of the measurement temperature. This is seen as distinguishing with regard to systems using a refresh or outgassing of the sensing layer, where typically the layer temperature is allowed to drop back to the environmental temperature.

For the purpose of measuring sulfide compounds the metal oxide layer comprises preferably tin oxide, particularly tin oxide in form of nanoparticles. The tin oxide can further be doped with palladium, preferably with the doping being in the range between one and 4 weight percent.

The portable device can be a smart phone, a handheld or wearable computer, a laptop, an electronic reader, a tablet computer, a game controller, a pointing device, a photo or a video camera, or a computer peripheral. Its housing is typically a shell of metal, glass, or plastic material and can be assembled as a unibody or from several parts. Enclosed in the housing are typically processors, drivers for parts such as screens, antennae, cameras, microphones and speakers as well as batteries to provide power to the device and its parts. A screen is typically arranged as a part of the housing or mounted behind a transparent window of the housing.

The sensor is best integrated with CMOS circuitry for control and read-out onto a common substrate.

Another aspect of the invention is a method of controlling one or more heating elements for heating a metal oxide layer of a chemical sensor upon initialisation of a measurement according to a temperature profile including an activation period during which the layer temperature is above a measurement temperature prior to a measurement period during which the layer temperature is at the measurement temperature, and wherein a concentration value of a gas compound is derived from values of a parameter related a resistance of the metal oxide layer as registered during the measurement period.

The above and other aspects of the present invention together with further advantageous embodiments and applications of the invention are described in further details in the following description and figures.

### BRIEF DESCRIPTION OF THE FIGURES

FIG. 1A is a schematic perspective view of a metal oxide gas sensor according to an embodiment of the present invention;
FIG. 1B is schematic cross-section of the device of Fig. 1A;
FIG. 2 is a schematic measurement graph illustrating a measurement at four different levels of H2S concentrations in air;
FIG. 3 illustrates the effect of the activation period; and
FIG. 4 illustrates the cross-sensitivity between H2S, ethanol (EtOH) and humidity in air.

### DETAILED DESCRIPTION

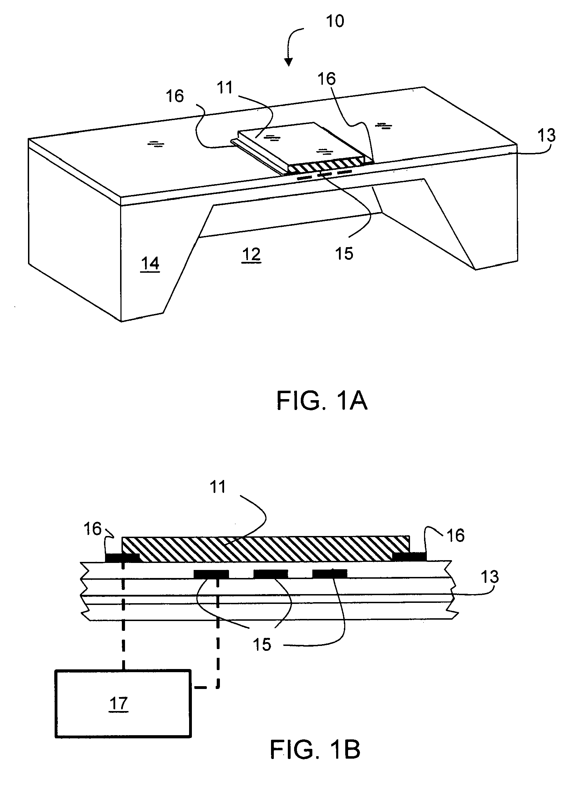

A gas sensor 10 - also referred to as chemical sensor or sensor - with a sensing layer 11 of metal oxide - also referred to as metal oxide layer or metal oxide - is shown in Figs 1A and 1B. The sensor is integrated with a CMOS circuitry (not shown) on a single chip. Layers 13 and handle layer 14 required for the CMOS circuit are etched away to form a cavity 12 at the location of the sensing layer 11. The remaining layers 13 form a thin membrane to support the actual sensor 10.

Embedded within the layers 13 are conducting elements - also referred to as heating elements 15-forming a heater to provide a local source of heat to heat the metal oxide 11 during operation of the sensor. The temperature can rise rapidly around the metal oxide layer 11, while the thicker part of chip reacts due to its thermal inertia with a slower rise of temperature. By controlling the heater accordingly, the metal oxide can be activated for a measurement and regenerated afterwards.

The metal oxide layer 11 is contacted by two conductive electrodes 16 and hence acts as a resistor. In the presence of an analyte its resistance changes thereby providing a measure of the concentration of the analyte in the immediate vicinity of the metal oxide layer.

Both the conductive electrodes 16 and the heating elements 15 are connected to a control unit 17, which can be part of the CMOS circuitry arranged on the same handle layer 14 acting as substrate as the metal oxide layer 11. The function of the control unit 17 is to control the amount of electrical power flowing into the heating elements 15 and to perform and register measurements to determine the changes in the resistance of the metal oxide layer 11.

When initialising a measurement, the control unit 17 raises the temperature of the metal oxide layer 11 first to an activation temperature for a predetermined period of time, herein referred to as the activation period. After the activation period, the control unit 17 lets the temperature of the metal oxide layer 11 drop to a measurement temperature and maintains the metal oxide layer 11 at the measurement temperature for a duration herein referred to as measurement period. During the measurement period the resistance of the metal oxide layer 11 is measured and converted into a value representative of the concentration of, in the present example, hydrogen sulfide in the air above the metal oxide layer 11.

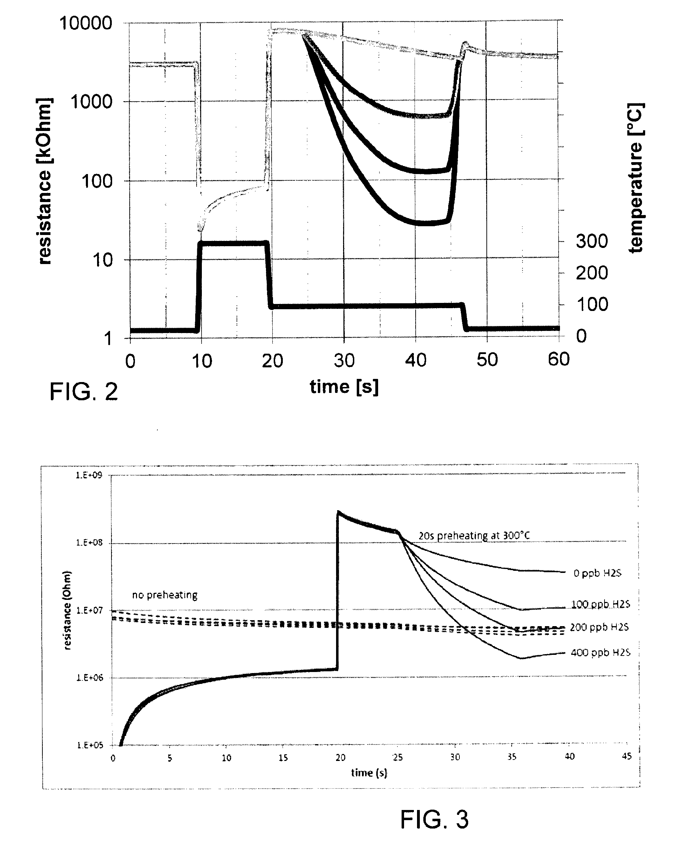

In FIG. 2 there is shown a schematic example illustrating a measurement as described above. The graph shows the resistance of a metal oxide layer in KOhms on the logarithmic scale and the temperature profile of the metal oxide layer in degrees Celsius (bottom graph) over the linear time axis from 0 to 60 seconds.

As shown, the measurement is initialised at 10 seconds, after which the temperature of the metal oxide layer is raised from room temperature to about 300°C as activation temperature for a period of 10 seconds. This period of 10 seconds is the activation period. At 20 seconds the temperature of the metal oxide layer drops to one hundred degrees Celsius as measurement temperature for about 27 seconds before going back to room temperature.

The period during which the temperature of one hundred degrees Celsius is maintained is the measurement period. At 25 seconds, i.e., five seconds into the measurement period the sampling of resistance values and hence of concentration values of hydrogen sulfide starts. The four branches of the upper resistance curve represent measurements at four different levels of hydrogen sulfide concentrations including zero (top branch). At the end of the measurement period it is possible to distinguish clearly between the four different levels of concentration.

A comparison between measurements without preheating or activation step and measurements with preheating or activation step is shown in FIG. 3. The figure shows the resistance measurements for four different gas concentrations of hydrogen sulfide, 0ppb, 100 ppb, 200 ppb and 400 ppb. The first group of measurements is performed without preheating at a measurement temperature of 85°C. The resulting signals are represented by the dashed lines and show very little variation with respect to the changing concentration of hydrogen sulfide. The second group of measurements is performed with a preheating of the sensing layer to an activation temperature of about 300°C. After the preheating period of about 20 seconds the sensing layer is allowed to cool down to a temperature of about 85°C. The resulting signals are represented by the solid lines and show a clear variation of the resistance with the gas concentration of hydrogen sulfide.

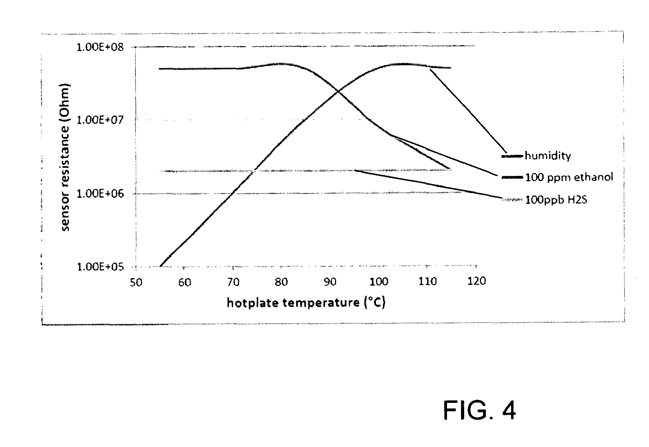

The temperature of the sensing layer during the measurement period is selected to minimize the cross sensitivity of the material of the sensing element to different components of the gas to be analyzed. A sensitivity plot of the resistance for a range of temperatures from 50 °C to 120 °C for humidity, hundred ppm ethanol, and one hundred ppb of hydrogen sulfide, respectively, is shown in FIG. 4. The resistance for humidity rises within most of this temperature range, while the resistance for ethanol begins to drop at about 80°C. The resistance for hydrogen sulfide remains essentially constant. From the plot shown in FIG. 4 the lowest cross sensitivity can be expected in the range from 85 to 90°C.

The similar behavior albeit at different temperature ranges can be expected of other gases such as carbon monoxide in the presence of humidity or ethanol.

While there are shown and described presently preferred embodiments of the invention, it is to be understood that the invention is not limited thereto but may be otherwise variously embodied and practised within the scope of the following claims.

## Claims

1. A chemical sensor (10) comprising at least one layer of a metal oxide (11) arranged between two electrodes (16), one or more heating elements (15) arranged to heat the layer of metal oxide (11), and a control unit (17) for controlling the heating elements (15), wherein the control unit (17) is adapted to control the one or more heating elements (15) to heat upon initialisation of a measurement the metal oxide layer (11) according to a temperature profile including an activation period during which the layer temperature is above a measurement temperature prior to a measurement period during which the layer temperature is at the measurement temperature, and wherein the control unit is adapted to (17) derive a concentration value of a gas compound from values of a parameter related to a resistance of the metal oxide layer (11) as registered during the measurement period.

2. The chemical sensor (10) according to claim 1, wherein the gas compound is selected from a group consisting of hydrogen sulfide, carbon monoxide and methane.

3. The chemical sensor (10) according to claim 1 or 2, wherein the gas compound is part of a sample of breath.

4. The chemical sensor according to any of the preceding claims, wherein a maximum temperature of the metal oxide layer (11) during the measurement period is in the range of 0°C to 200°C, in particular in the range between 50°C and 150°C.

5. The chemical sensor according to any of the preceding claims, wherein a maximum temperature of the metal oxide layer (11) during the activation period is in the range between 200°C and 400°C, in particular in the range between 250°C and 350°C.

6. The chemical sensor according to any of the preceding claims, wherein the control unit (17) is adapted to control the temperature profile such that the layer temperature does not drop below the measurement temperature minus 10% between the end of the activation period and the beginning of the subsequent measurement period.

7. The chemical sensor according to any of the preceding claims, wherein the control unit (17) is adapted to control the activation period and the subsequent measurement period to take place in a time window of less than one minute.

8. The chemical sensor according to any of the preceding claims, wherein a difference between the maximum temperature of the metal oxide layer (11) during the activation period and the maximum temperature of the metal oxide layer (11) during the measurement period is at least 50°C, in particular at least 100°C.

9. The chemical sensor according to any of the preceding claims, wherein the metal oxide layer (11) comprises tin oxide (SnO2).

10. The chemical sensor according to any of the preceding claims, wherein the metal oxide layer (11) comprises tin oxide doped with palladium.

11. A portable electronic device comprising a chemical sensor in accordance with any of the preceding claims.

12. A method of determining a parameter representing the concentration of a compound in a gas using a chemical sensor (10) with a metal oxide layer (11), comprising the steps of controlling heating one or more elements (15) for heating the metal oxide layer (11) of the chemical sensor (10) upon initialisation of a measurement according to a temperature profile including an activation period during which the layer temperature is above a measurement temperature prior to a measurement period during which the layer temperature is at the measurement temperature, and wherein a concentration value of a gas compound is derived from values of a parameter related to a resistance of the metal oxide layer (11) as registered during the measurement period.

13. The method of claim 12, wherein the temperature profile is such that the layer temperature is not significantly below the measurement temperature between the end of the activation period and the beginning of the subsequent measurement period.

14. The method of claim 12 or 13, wherein the gas compound is selected from a group consisting of hydrogen sulfide, carbon monoxide and methane.

15. The method of any of claims 12 to 14, wherein the gas compound is part of a sample of breath.

16. The method of any of claims 12 to 15, wherein a maximum temperature of the metal oxide layer (11) during the measurement period is in the range of 0°C to 200°C, in particular in the range between 50°C and 150°C.

17. The method of any of claims 12 to 16, wherein a maximum temperature of the metal oxide layer (11) during the activation period is in the range between 200°C and 400°C, in particular in the range between 250°C and 350°C.
